# DEMANDE DE BREVET EUROPEEN

(11) **EP 0 597 779 A1**
(43) Date de publication de la demande: **18.05.1994**
(21) Numéro de dépôt: 93420442.1
(22) Date de dépôt: 05.11.1993
(51) Int. Cl.: A61L 11/00

(54) **Machine pour le traitement de déchets médicaux**

(30) Priorité: 10.11.1992 FR 9213757
(71) Demandeur: SOCIETE NOUVELLE DES ETABLISSSEMENTS J. LAGARDE, F-26200 Montélimar (FR)
(72) Inventeur: Peyremorte, Jean-Pierre, F-26200 Montélimar (FR)
(74) Mandataire: Bratel, Gérard

(57) **Abrégé**

L'invention concerne notamment le traitement des déchets hospitaliers.

La machine comprend une enceinte (4,14) apte à être fermée de façon étanche, avec une trémie (4) munie d'une porte d'entrée (5), un déchiqueteur ou broyeur (10), une chambre inférieure (14) de réception et de stérilisation des déchets déchiquetés et broyés, et des moyens mécaniques (15) d'extraction de ces déchets au travers d'une porte de sortie (18). Ainsi, les déchets sont déchiquetés ou broyés à l'intérieur de la même enceinte (4,14), qui est reliée à des moyens d'injection de vapeur et à des moyens de mise sous vide.

## Description

L'invention concerne une machine pour le traitement de déchets médicaux, notamment de déchets hospitaliers, la machine comprenant une enceinte apte à être fermée de façon étanche, dans laquelle est introduite une charge de déchets à traiter, qui sont déchiquetés ou broyés à l'intérieur de cette enceinte maintenue fermée, et stérilisés à l'intérieur de la même enceinte maintenue fermée, la stérilisation des déchets déchiquetés ou broyés étant effectuée par injection de vapeur dans l'enceinte précitée, en alternance avec des mises sous vide de cette enceinte, avant que ladite enceinte soit rouverte pour extraire et évacuer les déchets, déchiquetés ou broyés et stérilisés. Le terme "traitement" englobe donc, dans le cadre de la présente invention, aussi bien des opérations de transformation mécanique des déchets en cause, notamment déchiquetage ou broyage, que des opérations visant à la stérilisation ou désinfection de ces déchets.

Les déchets hospitaliers nécessitent une destruction sur place, ou une stérilisation aussi complète que possible, qui les transforme en déchets assimilables à des déchets ménagers, et pouvant être évacués comme tels.

Si l'on exclut les procédés de destruction par incinération qui sortent du cadre de la présente invention, on connaît notamment des installations de traitement de déchets hospitaliers comprenant un dispositif de broyage en continu, suivi d'un "tunnel" de stérilisation, dans lequel les déchets avancent sous l'effet d'une vis. Ce genre d'installations se trouve décrit et illustré dans les documents DE-A-3938546, DE-U-9112202, EP-A-0277507, EP-A-0456237, EP-A-0476292 et WO-A-92/12738 (figure 3). Certaines réalisations décrites dans ces documents comportent un tunnel de stérilisation à deux tronçons successifs. Des portes ou sas intermédiaires étanches séparent, habituellement, le dispositif de broyage du tunnel de stérilisation et aussi, le cas échéant, les deux tronçons du tunnel de stérilisation.

Ainsi, les réalisations industrielles correspondantes restent des installations encombrantes et coûteuses, de sorte qu'en pratique elles peuvent seulement être communes à plusieurs hôpitaux ou cliniques, et que par conséquent elles ne suppriment pas la collecte et le transport des déchets hospitaliers, à moins d'être prévues elles-mêmes mobiles ce qui pose d'autres difficultés (installations montées sur des véhicules - voir les documents précités EP-A-0456237 et EP-A-0476292).

De plus, en raison de la subdivision de ces installations connues en deux ou trois enceintes par les portes ou sas intermédiaires, la vapeur de stérilisation injectée dans le tunnel de stérilisation n'atteint pas le dispositif de broyage. Ainsi, des moyens additionnels doivent être prévus pour stériliser le dispositif de broyage lui-même et les déchets éventuellement bloqués dans le dispositif de broyage, ces moyens additionnels n'intervenant qu'à la fin d'un cycle de fonctionnement.

Par le document précité WO-A-92/12738 (figures 1,2A et 2B), on connaît aussi une installation de stérilisation simplifiée, sans tunnel de stérilisation, dans laquelle s'effectuent, à l'intérieur d'une même enceinte unique, un broyage ou déchiquetage des déchets médicaux, ainsi qu'une stérilisation de ces déchets par chauffage et/ou injection de vapeur, suivi d'une mise sous vide. Toutefois, le dispositif se présente comme un tonneau de traitement, pourvu d'une seule porte d'accès qui sert aussi bien à l'introduction des déchets à traiter qu'à leur sortie après traitement. En fin de traitement, le déchargement des déchets stérilisés s'effectue par basculement du tonneau, aucun moyen mécanisé d'extraction n'étant prévu pour faire sortir les déchets. Il s'agit donc d'un dispositif seulement adapté au traitement discontinu de relativement faibles quantités de déchets hospitaliers, ce dispositif étant inapte à traiter de façon industrielle un flux continu et important de déchets.

Par ailleurs, d'une façon générale, les installations existantes de traitement des déchets hospitaliers ne sont pas équipées pour la décontamination des rejets liquides ou condensats, de sorte que la stérilisation effectuée reste incomplète.

La présente invention vise à remédier à l'ensemble des inconvénients précités, en fournissant une machine pour le traitement des déchets médicaux qui soit de conception très compacte et économique, tout en étant adaptée au traitement de quantités importantes de déchets grâce à une mécanisation appropriée permettant de maîtriser le flux de déchets traités, cette machine assurant par ailleurs une stérilisation complète, qu'il s'agisse des déchets eux-mêmes, du broyeur ou des condensats, par un processus efficace utilisant la vapeur et le vide.

A cet effet, la machine selon l'invention pour le traitement de déchets médicaux, appartenant au genre précisé en introduction, comprend une trémie de réception des déchets à traiter, munie d'une porte d'entrée à fermeture étanche, la trémie surmontant un déchiqueteur ou broyeur sous lequel est ménagée une chambre inférieure de réception et de stérilisation des déchets déchiquetés et broyés, avec des moyens mécaniques d'extraction de ces déchets au travers d'une porte de sortie à fermeture étanche, l'ensemble formé par la trémie, le déchiqueteur ou broyeur et la chambre inférieure formant ainsi une enceinte apte à être fermée de façon étanche, qui est reliée à des moyens d'injection de vapeur et à des moyens de mise sous vide.

Ainsi, cette machine assure le broyage ou déchiquetage des déchets, ainsi que leur stérilisation, dans un même volume donc avec un encombrement optimisé, tout en permettant, lors de l'ouverture des portes d'entrée et de sortie, un déchargement et un rechargement simultanés.

De plus, le fait que la trémie de réception des déchets non traités et le déchiqueteur ou broyeur soient intégrés à l'enceinte de stérilisation permet, en cas d'incidents mécaniques (tels que blocage du déchiqueteur ou broyeur par un objet métallique volumineux), de stériliser les déchets non déchiquetés. Une intervention humaine visant à retirer manuellement ces déchets est alors possible, sans risque de contamination. La stérilisation systématique de la trémie et du déchiqueteur ou broyeur, au cours du fonctionnement normal de la machine, permet aussi des interventions sans risques, pour l'entretien ordinaire de la machine.

Selon une caractéristique complémentaire, le fond de la chambre inférieure de réception et de stérilisation des déchets déchiquetés ou broyés est relié, de préférence par l'intermédiaire d'un filtre, à un pot de récupération des condensats, relié à une arrivée d'eau pour le refroidissement des condensats et à une arrivée de vapeur pour la stérilisation des condensats. Ainsi, ces condensats sont stérilisés et refroidis, avant d'être rejetés.

Avantageusement, les moyens de mise sous vide comprennent une pompe à vide avec un circuit d'aspiration, relié à l'enceinte précitée, sur lequel sont intercalés un condenseur et un filtre stérilisable à la vapeur. En outre, du côté du refoulement de la pompe à vide, on prévoit de préférence un séparateur lié à l'anneau liquide de la pompe à vide.

Grâce à ces diverses dispositions, la machine ne produit aucun rejet contaminé, et les déchets solides déchiquetés ou broyés et stérilisés, finalement obtenus, deviennent assimilables à des déchets ménagers, et peuvent être évacués comme tels.

Dans l'ensemble, la conception de cette machine assure donc une sécurité absolue, aussi bien pour les déchets et autres effluents finalement rejetés, que pour les opérateurs appelés à intervenir.

De toute façon, l'invention sera mieux comprise à l'aide de la description qui suit, en référence au dessin schématique annexé représentant, à titre d'exemple non limitatif, une forme d'exécution de cette machine pour le traitement de déchets médicaux :
Figure 1 est une vue de face d'une machine conforme à la présente invention ;
Figure 2 en est une vue en coupe verticale, suivant II-II de figure 1, avec indication des circuits.

La machine de traitement de déchets proprement dite, désignée dans son ensemble par le repère 1, est associée à un dispositif d'alimentation constitué, dans l'exemple illustré, par un élévateur-basculeur 2 installé sur le côté de la machine 1 et recevant des bacs roulants 3 remplis de déchets à traiter.

La machine 1 comporte, dans sa partie supérieure, une trémie de réception 4 dont la capacité correspond à une charge de déchets à traiter, donc à la capacité d'un bac 3. La trémie 4 est pourvue à son sommet d'une porte d'entrée 5, montée pivotante autour d'un axe horizontal 6 et conçue pour fermer de façon étanche l'entrée de cette trémie 4. La porte pivotante 5 supporte, en son centre, un vérin 7 qui actionne un plateau de poussée 8.

Au-dessous de la trémie de réception 4, à l'intérieur du bâti 9 de la machine 1, est disposé un déchiqueteur 10 qui, de manière connue en soi, comprend deux séries parallèles de disques coaxiaux 11 et 12, s'imbriquant les uns dans les autres. Les deux séries de disques 11 et 12 sont entraînées en rotation en sens opposés, autour de leurs axes horizontaux respectifs, à partir d'un moteur électrique 13.

Au-dessous du déchiqueteur 10 s'étend une chambre inférieure 14 de réception et de stérilisation des déchets déchiquetés, dans laquelle est montée tournante une vis d'extraction 15, entraînée en rotation autour de son axe horizontal 16 à partir d'un autre moteur électrique 17. La vis d'extraction 15 se prolonge en avant de la façade du bâti 9, jusqu'à proximité d'une porte de sortie 18 à fermeture étanche.

Un tapis sans fin 19, pour l'évacuation des déchets traités dans la machine 1, a son point de départ situé sous la porte de sortie 18.

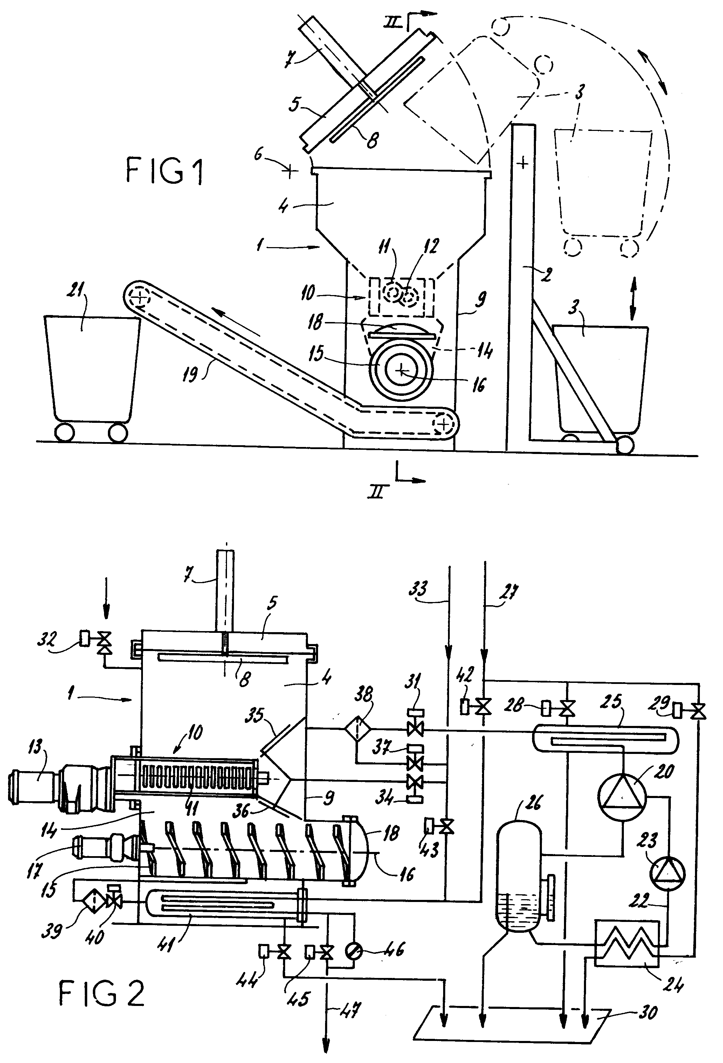

Le fonctionnement général de la machine, illustré notamment par la figure 1, est le suivant :

Un bac roulant 3, contenant une charge de déchets hospitaliers, est amené à la base de l'élévateur-basculeur 2 qui le saisit, l'élève jusqu'au sommet de la machine 1 et le vide dans la trémie 4, dont la porte d'entrée 5 est momentanément ouverte.

Pendant que le bac 3 vide est redéposé sur le sol par l'élévateur-basculeur 2, la porte d'entrée 5 de la trémie 4 est refermée de façon étanche. De même, la porte de sortie 18 est refermée de façon étanche.

Dès que les deux portes 5 et 18 sont refermées, le vérin 7 est commandé pour abaisser le plateau 8 qui pousse vers le bas les déchets en les répartissant, et le déchiqueteur 10 est mis en action pour réduire ces déchets sous forme de granulat. En même temps, le cycle de stérilisation commence. Le vide est créé, par une pompe à vide 20 (figure 2), dans toute l'enceinte étanche comprenant la trémie 4 et la chambre inférieure 14.

Les déchets déchiquetés parviennent dans la chambre inférieure 14 qui est sous vide. En fin de déchiquetage, la totalité des déchets rassemblés dans cette chambre 14 est soumise à une alternance d'injections de vapeur et de mises sous vide, assurant la stérilisation homogène de ces déchets et leur séchage.

En fin de cycle, l'enceinte constituée par la trémie 4 et la chambre inférieure 14 revient à la pression atmosphérique. Les portes d'entrée 5 et de sortie 18 sont ouvertes. La vis 15 est mise en marche pour extraire hors de la chambre inférieure 14 les déchets déchiquetés et stérilisés. Ces déchets, sous forme de granulat homogène, tombent sur le tapis 19 qui les évacue, par exemple en les délivrant dans un bac roulant 21 de réception des déchets stérilisés.

En se référant à la figure 2, on décrira maintenant les circuits, comprenant la pompe à vide 20 déjà citée, qui sont associés à la machine 1 notamment pour la stérilisation.

La pompe à vide 20 possède un anneau liquide 22 en circuit fermé, avec un circulateur 23 et un échangeur de refroidissement 24. Un condenseur 25 assure le bon fonctionnement de la pompe à vide 20. Le refoulement de cette pompe à vide 20 s'effectue dans un séparateur 26, la partie liquide étant refroidie et utilisée pour l'anneau liquide 22 de la pompe.

Une arrivée d'eau froide 27 est reliée, par l'intermédiaire d'une vanne 28, au condenseur 25 et, par l'intermédiaire d'une autre vanne 29, à l'échangeur de refroidissement 24 de l'anneau liquide 22. Les rejets d'eau, sortant du condenseur 25 et de l'échangeur 24, s'effectuent vers un égout symbolisé en 30.

Au-delà du condenseur 25, le circuit d'aspiration de la pompe à vide 20 est dirigé, en passant par une vanne 31 de mise sous vide, vers l'enceinte de la machine 1 composée de la trémie 4 et de la chambre 14. Une vanne casse-vide 32 est encore raccordée à cette enceinte.

Une arrivée de vapeur 33 est reliée, par l'intermédiaire d'une vanne 34 d'alimentation directe en vapeur, à des rampes 35 et 36 disposées respectivement dans la trémie 4 et dans la chambre 14 pour la distribution de vapeur. L'arrivée de vapeur 33 est encore reliée, par l'intermédiaire d'une vanne 37, à un filtre absolu 38 intercalé sur le circuit d'aspiration de la pompe à vide 20. Ainsi, les rejets gazeux passent par le filtre absolu 38, stérilisable à la vapeur.

Le fond de la chambre inférieure 14 est relié, par l'intermédiaire d'un filtre 39 et d'une vanne d'isolation 40, à un pot 41 de récupération des condensats. Le pot 41 est relié encore à l'arrivée d'eau 27 par l'intermédiaire d'une vanne 42, et à l'arrivée de vapeur 33 par l'intermédiaire d'une vanne 43. Grâce à ces dispositions, les condensats récupérés dans le pot 41 sont stérilisés à la vapeur en ouvrant d'abord la vanne 43, puis refroidis par circulation d'eau en ouvrant la vanne 42, avant d'être rejetés vers l'égout 30 en passant par une vanne 44 de vidange du pot 41.

Il est encore prévu, en aval du pot 41, une vanne 45 de sortie de l'eau de refroidissement des condensats, et un purgeur 46 de sortie de la vapeur de stérilisation des condensats, avec possibilité de récupération d'eau chaude en 47.

Dans l'ensemble, la machine 1 et les circuits associés évitent tout rejet à l'atmosphère de gaz ou liquides non stériles.

Toutes les températures requises pour la stérilisation sont contrôlées par des capteurs thermoélectriques, et toutes les pressions sont contrôlées par des capteurs de pression ; ces capteurs n'ont pas été représentés, pour la clarté du dessin.

Comme il va de soi, l'invention ne se limite pas à la seule forme d'exécution de cette machine pour le traitement de déchets médicaux qui a été décrite ci-dessus, à titre d'exemple ; elle en embrasse, au contraire, toutes les variantes de réalisation et d'application entrant dans le cadre des revendications annexées. C'est ainsi, notamment, que l'on ne s'éloignerait pas du cadre de l'invention par des modifications constructives, par exemple en remplaçant le plateau de poussée par un autre organe équivalent par sa fonction, ou par des adaptations apportées au détail des circuits, ou encore par une destination à des déchets non hospitaliers mais conservant un caractère médical, donc nécessitant le même traitement.

## Revendications

1. Machine pour le traitement de déchets médicaux, notamment de déchets hospitaliers, la machine comprenant une enceinte (4,14) apte à être fermée de façon étanche, dans laquelle est introduite une charge de déchets à traiter, qui sont déchiquetés ou broyés à l'intérieur de cette enceinte (4,14) maintenue fermée, et stérilisés à l'intérieur de la même enceinte (4,14) maintenue fermée, la stérilisation des déchets déchiquetés ou broyés étant effectuée par injection de vapeur dans l'enceinte précitée (4,14), en alternance avec des mises sous vide de cette enceinte, avant que ladite enceinte soit rouverte pour extraire et évacuer les déchets, déchiquetés ou broyés et stérilisés, caractérisée en ce qu'elle comprend une trémie (4) de réception des déchets à traiter, munie d'une porte d'entrée (5) à fermeture étanche, la trémie (4) surmontant un déchiqueteur ou broyeur (10) sous lequel est ménagée une chambre inférieure (14) de réception et de stérilisation des déchets déchiquetés ou broyés, avec des moyens mécaniques d'extraction (15) de ces déchets au travers d'une porte de sortie (18) à fermeture étanche, l'ensemble formé par la trémie (4), le déchiqueteur ou broyeur (10) et la chambre inférieure (14) formant ainsi une enceinte apte à être fermée de façon étanche, qui est reliée à des moyens d'injection de vapeur (33 à 36) et à des moyens de mise sous vide (20).

2. Machine pour le traitement de déchets médicaux selon la revendication 1, caractérisée en ce que la porte d'entrée (5) de la trémie (4) de réception des déchets à traiter supporte un vérin (7) prévu pour actionner un plateau de poussée (8), apte à être abaissé pour pousser les déchets vers le bas en les répartissant.

3. Machine pour le traitement de déchets médicaux selon la revendication 1 ou 2, caractérisée en ce que les moyens mécaniques d'extraction sont constitués par une vis d'extraction (15) montée tournante dans la chambre inférieure (14) de réception et de stérilisation des déchets déchiquetés ou broyés.

4. Machine pour le traitement de déchets médicaux selon l'une quelconque des revendications 1 à 3, caractérisée en ce que le fond de la chambre inférieure (14) de réception et de stérilisation des déchets déchiquetés et broyés est relié, de préférence par l'intermédiaire d'un filtre (39), à un pot (41) de récupération des condensats, relié à une arrivée d'eau (27,42) pour le refroidissement des condensats et à une arrivée de vapeur (33,43) pour la stérilisation des condensats.

5. Machine pour le traitement de déchets médicaux selon l'une quelconque des revendications 1 à 4, caractérisée en ce que les moyens d'injection de vapeur dans l'enceinte précitée comprennent, reliées à une arrivée de vapeur (33,34), des rampes (35,36) de distribution de vapeur disposées respectivement dans la trémie (4) et dans la chambre inférieure (14).

6. Machine pour le traitement de déchets médicaux selon l'une quelconque des revendications 1 à 5, caractérisée en ce que les moyens de mise sous vide comprennent une pompe à vide (20) avec un circuit d'aspiration, relié à l'enceinte précitée, sur lequel sont intercalés un condenseur (25) et un filtre (38) stérilisable à la vapeur.

7. Machine pour le traitement de déchets médicaux selon la revendication 6, caractérisée en ce que, du côté du refoulement de la pompe à vide (20), il est prévu un séparateur (26) lié à l'anneau liquide (22) de la pompe a vide (20).

8. Machine pour le traitement de déchets médicaux selon l'une quelconque des revendications 1 à 7, caractérisée en ce qu'il lui est associé un dispositif d'alimentation constitué par un élévateur-basculeur (2) recevant des bacs (3) remplis de déchets à traiter.
